# EUROPEAN PATENT APPLICATION

(11) **EP 4 091 948 A1**
(43) Date of publication of application: **23.11.2022**
(21) Application number: 22174021.0
(22) Date of filing: 18.05.2022
(51) Int. Cl.: B65B 31/04, A61L 2/00, A61L 2/26, B65B 55/02

(54) **STERILIZING PACKAGING UNITS**

(30) Priority: 18.05.2021 US 202163190093 P
(71) Applicant: Gerresheimer Glas GmbH, 40468 Düsseldorf (DE); STEVANATO GROUP S.P.A., 35017 Piombino Dese (PD) (IT)
(72) Inventor: REDEKER, Christian, 30890 Barsinghausen (DE); MAZZA, Fausto, 40125 Bologna (IT); PACE, Raffaele, 41121 Modena (IT)
(74) Representative: Fish & Richardson P.C.

(57) **Abstract**

The present disclosure describes a method of sterilizing packaging units (200) that includes packing a sterilization container (80) by receiving a rigid body (10) that includes a lower surface (12), an upper rim (14), and a plurality of side walls (16) that extend between the lower surface and the upper rim, wherein the upper rim defines an opening (22) to an interior space (20) of the body (10), arranging one or more packaging units (200) in the interior space of the body, covering and sealing the opening of the body with a rigid or semi-rigid lid (26), and engaging a valve body (32) arranged in a valve bore (30) that is in fluid communication with the interior space (20) of the body to move the valve body to an open position; exposing the interior space (20) of the body and the one or more packaging units (200) to a sterilant through the valve bore (30) until the one or more packaging units are sterilized; subjecting the interior space (20) of the body to a negative pressure differential; and engaging the valve body (10) to move the valve body to a closed position that seals the valve bore (30) and the interior space (20) of the body (10).

## Description

This disclosure relates to a method of sterilizing packaging units and a corresponding container.

Packaging units are used in various industries to store an end product, for example, a food product, a cosmetic product, or a pharmaceutical product. Packaging units that come into direct contact with such end products may be known as "primary packaging." Examples of primary packaging can include vials, cartridges, ampoules, bottles, or pre-fillable syringes. In some instances, primary packaging is sterilized before being filled with the end product. Generally speaking, sterilization processes use heat, chemicals, or radiation to kill microorganisms, such as bacteria or fungi. Primary packaging can sometimes be sterilized while still inside a secondary packaging that groups, protects, and labels the primary packaging.

EP 2 408 483 B1 describes a tray for vials or containers or for other instruments or devices, said tray comprising side walls, a bottom wall and a completely or partly open top surface defining an inside packaging area and an outside packaging area. At least one wall or top surface comprises an outlet to which is connected an outlet tube to let a gas pass through said outlet. Said outlet tube comprises a proximal end and a distal end. Said outlet tube comprises between the proximal end and the distal end a closing means made partly or completely of a shape memory material. Said closing means close the outlet tube. Said packaging optionally comprises a removable sheet or film placed on the tray top surface to isolate the inside packaging area from the outside packaging area.

Aspects of the present disclosure aim to alleviate problems associated with known containers.

According to a first general aspect of the present disclosure, a method of sterilizing packaging units includes packing a sterilization container by receiving a rigid body that includes a lower surface, an upper rim, and a plurality of side walls that extend between the lower surface and the upper rim, wherein the upper rim defines an opening to an interior space of the body, arranging one or more packaging units in the interior space of the body, covering and sealing the opening of the body with a rigid or semi-rigid lid, and engaging a valve body arranged in a valve bore that is in fluid communication with the interior space of the body to move the valve body to an open position; exposing the interior space of the body and the one or more packaging units to a sterilant through the valve bore until the one or more packaging units are sterilized; subjecting the interior space of the body to a negative pressure differential; and engaging the valve body to move the valve body to a closed position that seals the valve bore and the interior space of the body.

Engaging the valve body to move the valve body to the open position may include applying suction to the valve body, or vice versa. Engaging the valve body to move the valve body to the closed position may include applying pressure to the valve body, or vice versa.

Covering and sealing the opening of the body with a rigid or semi-rigid lid may additionally include placing the body and lid in at least one flexible, gas-permeable bag.

The rigid or semi-rigid lid may include the valve bore and a recess surrounding the valve bore, wherein the recess is configured to fully accommodate the valve body when the valve body is in the open position.

Exposing the interior space of the body and the one or more packaging units to a sterilant through the valve bore may include receiving a plurality of packed sterilization containers, stacking the plurality of sterilization containers with the lids and lower surfaces of adjacent containers facing one another, and placing the stacked sterilization containers in a sterilization chamber.

Each container may include a tracking indicator that is scanned before the container is exposed to the sterilant. The sterilant may be vaporized hydrogen peroxide.

The method may additionally include engaging the valve body to move the valve body from the closed position back to the open position, separating the lid from the body, removing the packaging units stored in the interior space of the body, and storing the body and the lid for reuse.

According to a second general aspect of the present disclosure, a sterilization container includes a rigid body that includes a lower surface, an upper rim, and a plurality of side walls that extend between the lower surface and the upper rim, wherein the upper rim defines an opening to an interior space of the body; a rigid or semi-rigid lid that covers the opening of the body; a gasket arranged between the body and the lid; and a valve body arranged in a valve bore that is in fluid communication with the interior space of the body, wherein the valve body is moveable between an open position and a closed position in which the valve body closes the valve bore and isolates the interior space of the body.

The rigid or semi-rigid lid may include the valve bore. The lid may additionally include a recessed portion that surrounds the valve bore and is configured to fully accommodate the valve body when the valve body is in the open position.

The lid may include one or more clips that releasably engage the upper rim or sidewall of the body. In some instances, the gasket may be integrally formed with the lid. The valve body may include a substantially planar top surface.

These and other embodiments described herein may provide one or more of the following benefits. The sterilization method and container may eliminate flexible sheets of porous material that are often used as a lid for sterilization containers. Such flexible lids may be prone to particle generation that can contaminate the packaging units and must be disposed after a single use. At the same time, the form factor of the sterilization containers remains largely the unaffected. The sterilization method may be conducted as an in-line process, as the sterilization container maintains sterility. In-line sterilization processes may be flexible and may also reduce the need to outsource sterilization operations. The valve body may act as a physical barrier that maintains sterility of the packaging units while simultaneously serving as a visual indicator that the contents of the container are sterilized.

Certain embodiments will now be described, by way of example only, with reference to the accompanying drawings, in which:
Figure 1 shows a schematic flowchart of a method according to the present disclosure;
Figure 2 shows a schematic cross-sectional view of a body that can be used in the method of Figure 1;
Figure 3 shows the arrangement of one or more packaging units in the body of Figure 2;
Figure 4 shows an example of a sterilization container according to the present disclosure;
Figure 5 and 6 show enlarged views of the sterilization container of Figure 4; and
Figures 7A and 7B show schematic cross-sectional views of a further valve body according to the present disclosure.

Like reference numbers and designations in the various drawings indicate like elements.

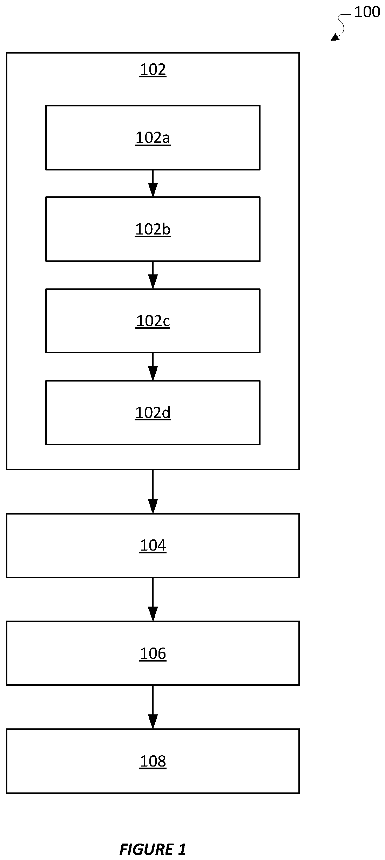

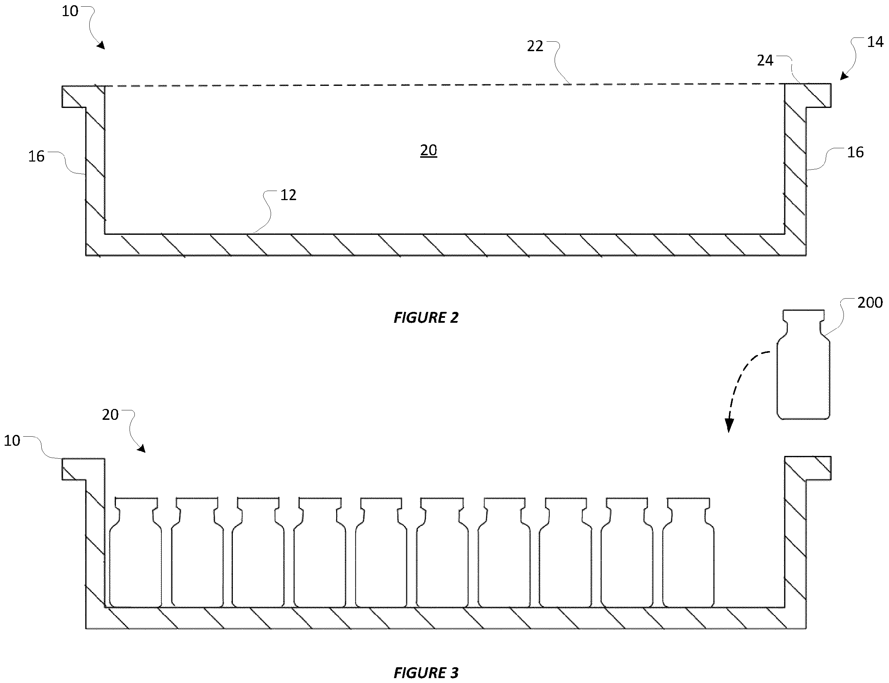

Figure 1 shows a schematic flowchart of a method 100 according to the present disclosure. The method 100 includes packing 102 a sterilization container by receiving 102a a rigid body that includes a lower surface, an upper rim, and a plurality of side walls that extend between the lower surface and the upper rim, wherein the upper rim defines an opening to an interior space of the body, arranging 102b one or more packaging units in the interior space of the body, covering and sealing 102c the opening of the body with a rigid or semi-rigid lid, and engaging 102d a valve body arranged in a valve bore that is in fluid communication with the interior space of the body to move the valve body to an open position. The method 100 further includes exposing 104 the interior space of the body and the one or more packaging units to a sterilant through the valve bore until the one or more packaging units are sterilized, subjecting 106 the interior space of the body to ad negative pressure differential, and engaging 108 the valve body to move the valve body to a closed position that seals the valve bore and the interior space of the body. Details and optional aspects of the method 100 are described below in reference to Figures 2 to 7.

In the present disclosure, the term "negative pressure differential" when applied to a confined volume, means that the difference between the pressure value inside the confined volume and the pressure at the external volume surrounding the confined volume is negative.

In the present disclosure, the term "semi-rigid" indicates a body that is stiff and solid, but not inflexible, i.e., is able to flex. In contrast, "rigid" may be used to indicate a body that is formed of materials that do not allow the body to easily bend, flex, or deform.

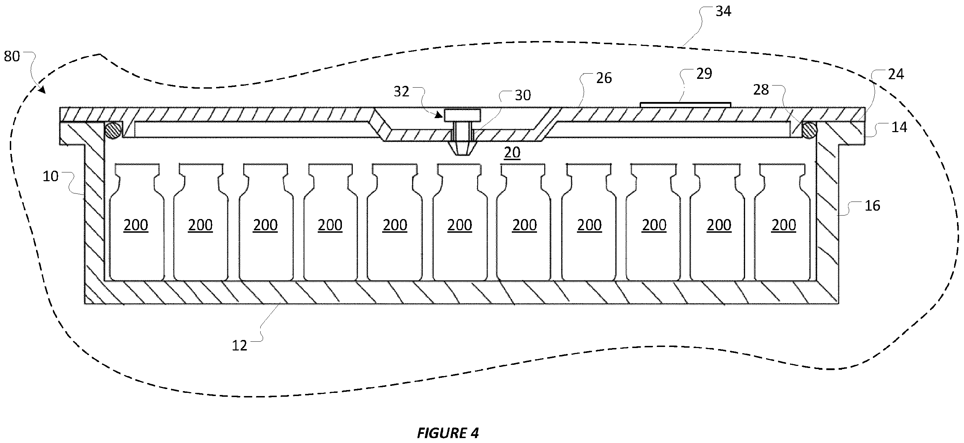

Figure 2 is a schematic cross-sectional view of a rigid body 10 of a container 80 (Figure 4). The body 10 and the container 80 can be used in the method 100 of Figure 1. The body 10 may be referred to as a "tub" and can be used as secondary packaging for primary packaging in the food, cosmetic, or pharmaceutical industries. Although the expression "primary packaging" can encompass vials, cartridges, ampoules, bottles, and syringes to name a few examples, the following description will refer to "vials" for all types of primary packaging.

The body 10 includes a lower surface 12, an upper rim 14, and side walls 16 that extend between the lower surface 12 and the upper rim 14. The lower surface 12 can be a flat planar surface that rests on a table or worktop (not shown). The lower surface 12 can have a rectangular shape with two long edges and two short edges. Accordingly, the side walls 16 can include an opposing pair of long side walls and an opposing pair of short side walls. All four side walls 16 extend in the vertical direction to connect the lower surface 12 and the upper rim 14. Together, the lower surface 12 and the side walls 16 define an interior space 20 of the body 10 that can receive, e.g., a set of vials to be filled with a pharmaceutical product (Figure 3).

The upper rim 14 defines an opening 22 to the interior space 20 of the body 10. As illustrated in Figure 2, the rim 14 can include a lip or flange 24 that encloses the opening 22 to the interior space 20. The flange 24 can extend substantially parallel to the lower surface 12. As illustrated, the flange 24 also extends orthogonally to the side walls 16; however, in some cases, the side walls 16 may also form an obtuse angle with respect to the lower surface 12.

As shown by the dashed arrow in Figure 3, one or more packaging units, e.g., vials 200, can be arranged in the interior space 20 of the body 10. Prior to arranging the vials 200 in the body 10, the vials 200 may be washed and subjected to a depyrogention process.

There are many ways of arranging or loading the vials 200 in the body 10. For example, the vials 200 can be placed in the body 10 individually, without any kind of supporting structure. In some instances, it may be necessary to keep the vials 200 from touching each other, e.g., to prevent scratches. In this case, the opening 22 can be sized to accommodate a support tray or "nest" that supports the vials 200 within the interior space 20 of the body 10 (not shown). The support tray can include an array of recesses that are each sized to receive a vial 200. The support tray can ensure that the vials are securely supported in an upright position and do not come into contact with one another. For example, an empty support tray can be inserted through the opening 22 of the interior space 20. Individual vials 200 can be arranged in the respective openings of the support tray.

Once the vials 200 have been arranged inside the body 10, a rigid or semi-rigid lid 26 is used to cover the opening 22 of the body 10, as shown in Figure 4. For example, the body 10 and the lid 26 may each be a monolithic part formed of molded plastic material.

As shown in Figure 4, the lid 26 is designed to seat against the flange 24 of the upper rim 14. A flexible gasket 28 is arranged between the lid 26 and an inner vertical wall of the upper rim 14 to establish a seal between the body 10 and the lid 26. The lid 26 and the upper rim 14 can include latches or clips to secure the body 10 to the lid 26 and compress the gasket 28. Although the gasket 28 and lid 26 are shown as separate parts in Figure 4, the lid 26 may include a groove for seating the gasket 28. In some cases, the gasket 28 and the lid 26 can be formed integrally, e.g., using insert molding techniques.

The lid 26 can include one or more tracking indicators 29 that are scanned before the container 80 and its contents are exposed to sterilant. Although the tracking indicator 29 is schematically shown by a raised surface, the tracking indicator 29 may also be etched into a top surface of the lid 26. Examples of tracking indicators 29 may include barcodes or QR codes that may be affixed by labels or etched into the material of the lid 26. Although the tracking code 29 is shown on the lid 26 in Figure 4, the tracking code 29 may also be located elsewhere on the body 10, e.g., on one of the side walls 16.

The container 80 also includes a valve bore 30 that is in fluid communication with the interior space 20 of the body 10. In the illustrated example, the valve bore 30 is formed in the lid 26. In some instances, the valve bore 36 may be formed in one of the side walls 16 or even the lower surface 12. Since the opening 22 of the body 10 is sealed by the lid 26 and the gasket 28, the valve bore 30 forms a single entrance for gases to enter and exit the interior space 20 of the body 10.

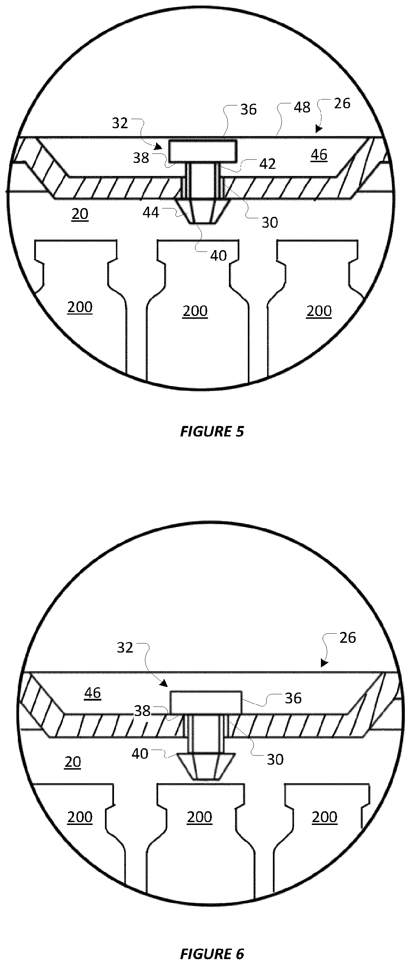

A valve body 32 is arranged in the valve bore 30 and is moveable between an open position shown in Figures 4 and 5 and a closed position shown in Figure 6. In the closed position, the valve body 32 closes, i.e., obstructs, the valve bore 30 and isolates the interior space 20 of the body 10. Details of the valve body 32 are described below in reference to Figures 4 to 6.

In some instances, the container 80 may be placed in at least one flexible, gas-permeable bag 34 after the lid 26 has been attached to the body 10. The bag 34 may be made of a porous plastic material that is permeable to sterilant but not to liquids, e.g., Tyvek^{®}. The bag 34 may protect the container 80 and its contents during the sterilization process. Since the bag 34 is made of flexible material, the valve body 32 can be manipulated through the bag, i.e., while the container 80 is inside. In some instances, the method 100 of Figure 1 can be implemented without the use of such a bag 34.

Figure 5 is an enlarged partial view of the valve bore 30 and valve body 32 of Figure 4. As illustrated, the valve body 32 includes a first enlarged end 36 with a sealing surface 38 and a second enlarged end 40. The first and second enlarged ends 36, 40 are connected by a valve stem 42 that extends through the valve bore 30. The valve body 32 may also include one or more passageways 44 that extend along the stem 42 and the second enlarged end 40 and allow gases to pass through the valve bore 30. The valve body 32 may be a monolithic part made of flexible material, e.g., silicone. In other embodiments, the valve 32 may have a different appearance than the one shown in Figures 4 to 6.

The lid 26 may include a recess 46 that surrounds the valve bore 30 and fully accommodates the valve body 32 when the valve body 32 is in the open position shown in Figure 5. In this disclosure, "fully accommodates" means that the valve body 32 does not protrude beyond a top surface 48 of the lid 26 when the valve body 32 is in the open position. The recess 46 allows the containers 80 to be stacked on top of one another for transport and storage, for example.

In order to move the valve body 32 into the open position shown in Figure 5, the valve body 32, e.g., the first enlarged end 36 is engaged. For example, suction may be applied to a top surface of the first enlarged end 36. In some cases, the first enlarged end 36 can be gripped by its peripheral surface and gently pulled in an upward direction. In some instances, the valve body 32 may be moved into this open position before the lid 26 is attached to the body 10 of the container 80.

Once in the open position shown in Figures 4 and 5, the interior space 20 of the body 10 and the vials 200 are exposed to sterilant through the valve bore. For example, the packed container 80 can be placed in a sterilization chamber that is filled with a chemical sterilant, such as vaporized hydrogen peroxide. Other gaseous sterilants, such as ethylene oxide (EtO) can also be used. At this point, there is no pressure differential inside and outside the container 80. After a certain exposure duration (e.g., 3 to 4 hours), the gases are evacuated from the sterilization chamber.

The valve body 32 is then moved to a closed position that seals the valve bore 30 and the interior space 20 of the body 10, as shown in Figure 6. For example, the container 80 can exit the sterilization chamber into a sterile area downstream of the chamber. At this point, the valve body 32 may still be in the open position. The container 80 can be placed in a flexible, gas-permeable bag 34, as described above. A robot arm can be used to place the container 80 on a flat surface and exert pressure on the lid 26 from outside of the bag 34, flexing the lid 26 inwards. The robot arm may apply further pressure onto a central part of the lid 26 where the valve body 32 is located. In the event that no bag 34 is used, the robot arm may include a suction unit that further increases the pressure differential before closing the valve body 32.

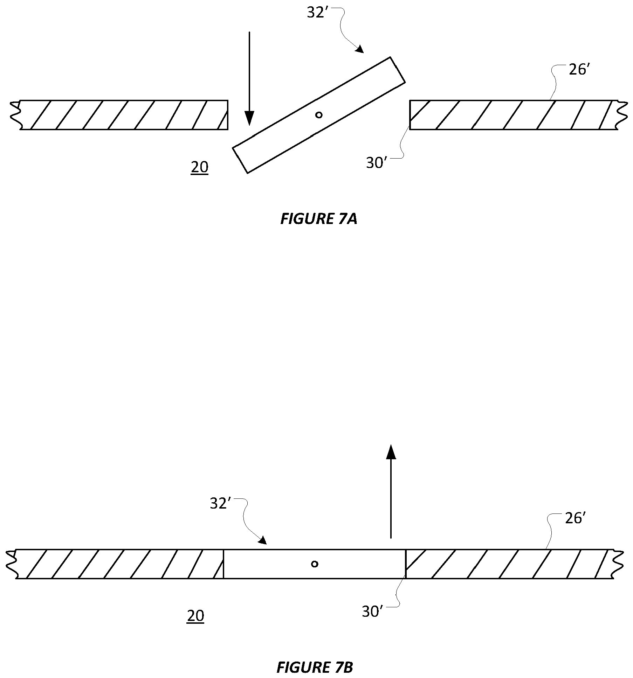

In the closed position, the sealing surface 38 of the first enlarged end 36 is seated against a top surface of the recess 46 (or the lid 26 if no recess 46 is present). The valve body 32 can be moved to the closed position by applying downward pressure on the first enlarged end 36, for example. In contrast, Figure 7A schematically depicts a valve body 32' that can be moved to the open position by applying downward pressure that pivots the valve body 32' to an open position. In Figure 7B, the valve body 32' can be moved to the closed position by applying suction to the open valve body 32'. The valve body 32' depicted in Figures 7A and 7B can be arranged in a lid 26' that does not include a recess 46, such as the one shown in Figures 5 and 6.

As the valve body 32, 32' is moved to the closed position, gases are further evacuated through the valve bore 30, 30', subjecting the interior space 20 to a negative pressure differential. The valve body 32, 32' acts as a barrier between the sterile interior space 20 and the non-sterile environment outside the container 80. The closed position 32, 32' of the valve body 32, 32' may serve as a visual indicator that the contents of the container 80 are sterilized.

Once the vials 200 are ready for further use, they may be unpacked from the container 80. For example, the container 80 may be unpacked under clean room conditions before the vials 200 are filled. In this case, the valve body 32 may be engaged to move the valve body 32 from the closed position (Figure 6) back to the open position (Figures 4 and 5). The movement of the valve body 32 may release the negative pressure differential and enable the lid 26 to be opened. After the vials 200 are removed from the body 10, the lid 26 and the body 10 may be stored for reuse, i.e., for a subsequent packing and sterilization process.

Although Figures 2 to 6 each show an individual container 80, the method 100 may include receiving multiple packed sterilization containers 80. The containers may be stacked with the lids 26 and lower surfaces 12 of adjacent containers 80 facing one another and placed in a sterilization chamber in the stacked configuration.

A number of embodiments have been described. Nevertheless, numerous alternative embodiments within the scope of the claims will be readily appreciated by those skilled in the art. The presently described embodiments are not to be taken as limiting the scope of the invention.

## Claims

1. A method of sterilizing packaging units, comprising:
packing a sterilization container by
receiving a rigid body that comprises a lower surface, an upper rim, and a plurality of side walls that extend between the lower surface and the upper rim, wherein the upper rim defines an opening to an interior space of the body,
arranging one or more packaging units in the interior space of the body, covering and sealing the opening of the body with a rigid or semi-rigid lid, and
engaging a valve body arranged in a valve bore that is in fluid communication with the interior space of the body to move the valve body to an open position;
exposing the interior space of the body and the one or more packaging units to a sterilant through the valve bore until the one or more packaging units are sterilized;
subjecting the interior space of the body to a negative pressure differential; and
engaging the valve body to move the valve body to a closed position that seals the valve bore and the interior space of the body.

2. The method according to claim 1, wherein engaging the valve body to move the valve body to the open position comprises applying suction to the valve body.

3. The method according to claim 1 or 2, wherein engaging the valve body to move the valve body to the closed position comprises applying pressure to the valve body.

4. The method according to claim 1, wherein engaging the valve body to move the valve body to the open position comprises applying pressure to the valve body.

5. The method according to claim 4, wherein engaging the valve body to move the valve body to the closed position comprises applying suction to the valve body.

6. The method according to any one of claims 1 to 5, wherein covering and sealing the opening of the body with a rigid or semi-rigid lid further comprises placing the body and lid in at least one flexible, gas-permeable bag.

7. The method according to any one of the preceding claims, wherein the rigid or semi-rigid lid comprises the valve bore and a recess surrounding the valve bore, wherein the recess is configured to fully accommodate the valve body when the valve body is in the open position.

8. The method according to claim 7, wherein exposing the interior space of the body and the one or more packaging units to a sterilant through the valve bore comprises:
receiving a plurality of packed sterilization containers;
stacking the plurality of sterilization containers with the lids and lower surfaces of adjacent containers facing one another; and
placing the stacked sterilization containers in a sterilization chamber.

9. The method according any one of the preceding claims, wherein each container comprises a tracking indicator that is scanned before the container is exposed to the sterilant.

10. The method according to any one of the preceding claims, wherein the sterilant is vaporized hydrogen peroxide.

11. The method according to any one of the preceding claims, further comprising:
engaging the valve body to move the valve body from the closed position back to the open position;
separating the lid from the body;
removing the packaging units stored in the interior space of the body; and
storing the body and the lid for reuse.

12. A sterilization container comprising:
a rigid body that comprises a lower surface, an upper rim, and a plurality of side walls that extend between the lower surface and the upper rim, wherein the upper rim defines an opening to an interior space of the body;
a rigid or semi-rigid lid that covers the opening of the body;
a flexible gasket arranged between the body and the lid; and
a valve body arranged in a valve bore that is in fluid communication with the interior space of the body, wherein the valve body is moveable between an open position and a closed position in which the valve body closes the valve bore and isolates the interior space of the body.

13. The container according to claim 12, wherein the rigid or semi-rigid lid comprises the valve bore.

14. The container according to claim 13, wherein the lid comprises a recessed portion that surrounds the valve bore and is configured to fully accommodate the valve body when the valve body is in the open position.

15. The container according to any one of claims 12 to 14, wherein the lid comprises one or more clips that releasably engage the upper rim or sidewall of the body.

16. The container according to any one of claims 12 to 14, wherein the gasket is integrally formed with the lid.

17. The container according to any one of claims 12 to 16, wherein the valve body comprises a substantially planar top surface.
